# EUROPEAN PATENT APPLICATION

(11) **EP 4 241 803 A1**
(43) Date of publication of application: **13.09.2023**
(21) Application number: 22161376.3
(22) Date of filing: 10.03.2022
(51) Int. Cl.: A61M 5/142, A61M 5/315, A61M 5/172, A61M 5/14, A61M 5/168

(54) **DRUG DELIVERY DEVICE**

(71) Applicant: TecMed AG, 3400 Burgdorf (CH)
(72) Inventor: Buri, Thomas, 3400 Burgdorf (CH); Streit, Ursina, 3422 Kirchberg (CH); Hulliger, Manuel, 4563 Gerlafingen (CH)
(74) Representative: Meier Obertüfer, Jürg

(57) **Abstract**

The present invention is concerned with a drug delivery device with an electric brushless DC or stepper motor. A novel approach to motor control uses the micro-stepping technique in combination with over-revving the rotor or commutation of the stepper motor and reversing the rotor for a small number of microsteps with each delivery cycle. This improvement has the effect of keeping the elasticity of the drive train behind the plunger in a more relaxed state after completing a delivery cycle, providing better control of the movement of the rotor. Loss of steps can be avoided for a wide range of friction or speed situations, improving the accuracy of drug delivery and making tracking of the rotor position and detecting loss of steps a much improved parameter to detect blockage or occlusion. The improved controllability allows to substantially increase the motor speed for delivery, resulting in an optimum of energy efficiency while still ensuring a most reliable occlusion detection.

## Description

### FIELD OF THE INVENTION

The present invention relates to drug delivery devices, in particular to infusion pumps or injectors with an electric brushless DC or stepper motor. A novel approach to motor control provides a design with improved energy efficiency, improved delivery characteristics and more reliable occlusion detection.

### BACKGROUND OF THE INVENTION

A variety of diseases exist that require regular treatment by subcutaneous administration of a medicament, and a number of delivery devices have been developed to support a patient in accurately and controllably delivering an amount of drug in a self-administration process. Drug delivery devices include injection devices that are removed from the site of application after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Common to all devices for subcutaneous drug delivery is a reservoir to store the fluid medicament, and a fluid path to bring the drug out of the device and into the subcutaneous tissue of a patient. In a majority of injecting or infusion devices the reservoir has a plunger which is mechanically advanced by an actuation assembly - in this case usually a plunger rod - to drive the fluid out of the reservoir into the fluid path and towards the patient. A typical driving mechanism has a motor driving a rotation which is translated into a linear movement of the plunger rod, for example a driving nut rotated by a gear box at the output of the motor in combination with a rotationally fixed rod with an inner thread. In practice, the mechanical drive train between the motor and the plunger inherently includes a certain elasticity, distributed over all elements involved, like motor bearings, the gearbox, driving thread, plunger and cartridge fixation. In a plunger-driven drug delivery system, the accuracy of drug delivery is defined by the accuracy of plunger movement. Just like elasticity, friction is distributed all along the drive train, which in combination greatly affects the delivery accuracy and adds complexity to motor control and supervision. The effect of elasticity is most obvious in case of a blockage or occlusion of the fluid path at the output of the infusion device, resulting in a delay of fault detection. But, in the inavoidable presence of friction, even during normal operation an elastic drive train is much more difficult to control than a rigid one.

In delivery devices for subcutaneous drug application the motor is operated intermittently, using discrete delivery cycles to control the drug delivery. Brushless DC motors or stepper motors are often used to generate a predefined number of angular steps at the output for every delivery cycle. This provides a well controllable basis to realise discrete movements of the plunger in the drug reservoir.

During application of an infusion system, a considerable amount of time passes between delivery cycles, typically several minutes or even more. During this time the mechanical system is subject to all sorts of changes in environmental conditions - from a change in ambient temperature to experiencing a fall on a floor - which are particularly difficult to control if elasticity is involved. Achieving highly accurate drug delivery and reliable occlusion detection in a system with intermittent operation of an inherently elastic drive train is a mayor challenge for mobile, battery-driven devices where energy-efficiency is an important requirement, and compact form factors do not allow for big tolerances of any parameter involved.

A first known approach for optimising drive control in such a system is to optimise motor control for energy-efficient normal operation. This means increasing the motor speed to keep the periods of activity short and maximise time in a standby or sleep mode where only a minimum of components is powered. For power efficient high-speed operation, such a stepper motor control will apply a predefined ramp to accelerate and deccelerate, to cater for the physical inertia of the drive train, and also to minimise acoustic noise. Micro-stepping is a technique well-known to the skilled person to realise such a motor control.

WO-2005/093533-A1 (T. Allen et al, 2005) describes a motor control for infusion systems optimised for energy efficient normal operation using micro-stepping. In this example, optimisation has a focus on controlling the electrical current, which is achieved by introducing micro-steps and apply a ramped driving voltage to the motor rather than just switching a constant voltage between driving pins.

A second known approach for optimising drive control in a system with an inherently elastic drive train is directed at fast and reliable detection of blockage and occlusion. A quick occlusion detection can greatly improve the accuracy subcutaneous drug delivery, where occlusions always need to be considered.

EP-3213785-A1 (Moberg, 2007) describes a motor control for infusion systems optimised for fast and reliable occlusion detection. Again, motor current is used a a key parameter to detect friction in the drive train and decide whether an occlusion is present or not.

WO12040528 A1 (Smith, 2011) improves delivery accuracy of a drug delivery system with an elastic drive train by reversing the motor and partially retracting the plunger rod to relax the elasticity at a zero position of the plunger. The retraction may also be applied if a blockage or occlusion has been detected.

While there is no direct connection between energy-efficient intermittent drug delivery and methods for fast and reliable occlusion detection, an optimum motor control for such a delivery system with inherent elasticity will have to address both issues. A combined optimum hence consists of a reliable, smooth movement and an occlusion error reported immediately after an occlusion has occurred.

US-2013253420-AA describes a motor control for infusion systems combining both approaches by running an intermittent drug delivery, by detecting physical steps lost due to any kind of friction, and by reporting an occlusion condition based on lost motor steps. Micro-stepping is used to minimise energy consumption for normal drug delivery. While applying this technology, missed motor steps are used as a criterium to adjust the driving power in case of increasing friction. The same parameter, when exceeding a predefined threshold, indicates a blockage or occlusion.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to provide a drug delivery device with an improved motor control to achieve highly accurate drug delivery and reliable occlusion detection at optimum energy efficiency in a system with intermittent operation of a potentially elastic drive train.

This objective is achieved by using the micro-stepping technique in combination with over-revving the rotor or commutation of the stepper motor and reversing the rotor or commutation for a small number of steps or micro-steps with each delivery cycle. This novel approach has the effect of keeping the elasticity of the drive train behind the plunger in a more relaxed state after completing a delivery cycle, providing better control of the movement of the rotor. Loss of steps can be avoided for a wide range of friction or speed situations, making tracking of the rotor position and detecting loss of steps a much improved parameter to detect blockage or occlusion. Avoiding step loss rather than just compensating it when step loss occurs is a radical change of paradigm leading to improved control which may analogously be applied to any kind of electric motor. The improved controllability allows not only to improve the accuracy of delivery for small amounts of drug, but also to substantially increase the motor speed for delivery, resulting in an optimum of energy efficiency while still ensuring a most reliable occlusion detection.

A mobile or wearable drug delivery device is provided comprising an electric motor with a rotor at the output; as well as an electronic control unit configured to control the commutation or rotation of the rotor and to intermittently advance an angular position of the rotor by a number of steps to convey a predefined amount of drug over a plurality of delivery cycles. The electronic control unit is adapted to over-revving, for each of a plurality of delivery cycles, the commutation or rotation of the rotor by a predefined number of over-revving steps in excess of the number of angular steps or micro-steps defined for said delivery cycles. The electronic control unit may further be adapted to reverse the commutation or rotation of the rotor, after over-revving, by substantially the same number of over-revving steps or micro-steps in an opposite direction. The electronic control unit may include means to measure or estimate any number of supervision parameters like motor drive current, back EMF, rotor position, rotor speed, time to reach a target position, loss of steps, plunger position or drive train friction. Such a parameter or plurality of parameters may be compared with a predefined reference value to detect an error condition. Typical error conditions include underdelivery, blockage of the motor, or occlusion of a fluid path at an output of the drug delivery device.

The drug delivery device may be a tubeless patch infusion pump attachable to the skin of a patient by means of an adhesive layer, for subcutaneous delivery of insulin through a cannula integrated into the pump over a period of more than 48 hours. Alternatively, the delivery device is not a tubeless patch infusion pump attachable to the skin of a patient by means of an adhesive layer, for subcutaneous delivery of insulin through a cannula integrated into the pump over a period of more than 48 hours. For instance, the alternative delivery device may be patch injector attachable to the skin of a patient by means of an adhesive layer, for subcutaneous delivery of a drug other than insulin over a period of less than 48 hours. The alternative delivery device may be a wearable insulin pump or a handheld injection pen devoid of an adhesive layer for attaching to the skin of a patient.

Such an improved delivery device implements a method of controlling an electric motor in a drug delivery device, whereby the method includes over-revving the commutation or rotation of the rotor by a predefined number of over-rewing steps. The method may further include reversing the commutation or target angular position of the rotor, after over-revving, by substantially the same number of over-revving steps in an opposite direction.

The method of controlling a motor using a combination of micro-stepping and over-revving according to the present invention may be applied, with or without subsequent reversing of the rotor, to a variety of embodiments, wherever energy efficiency and reliable control of rotor movement can be an advantage.

### BRIEF DESCRIPTION OF THE DRAWINGS

The subject matter of the invention will be explained in more detail in the following text with reference to preferred exemplary embodiments which are illustrated in the attached drawings, in which:

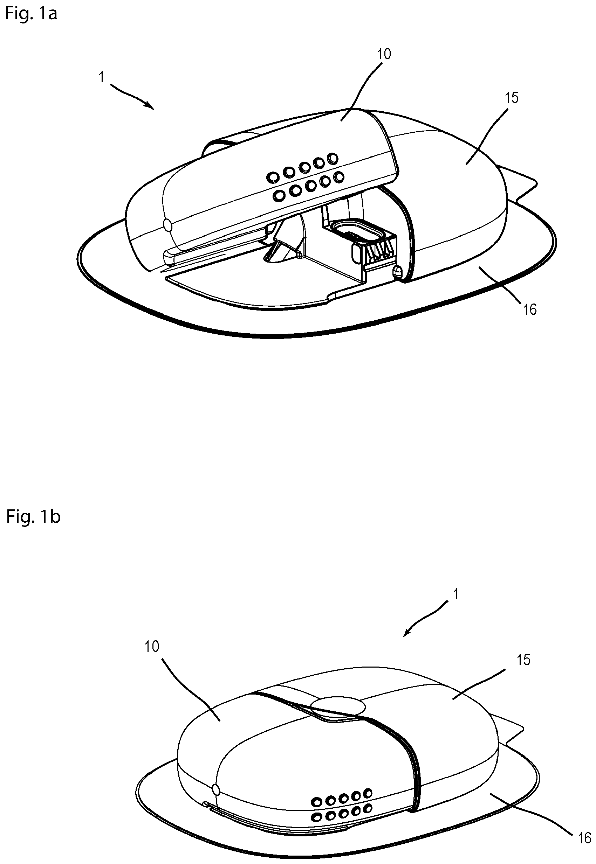

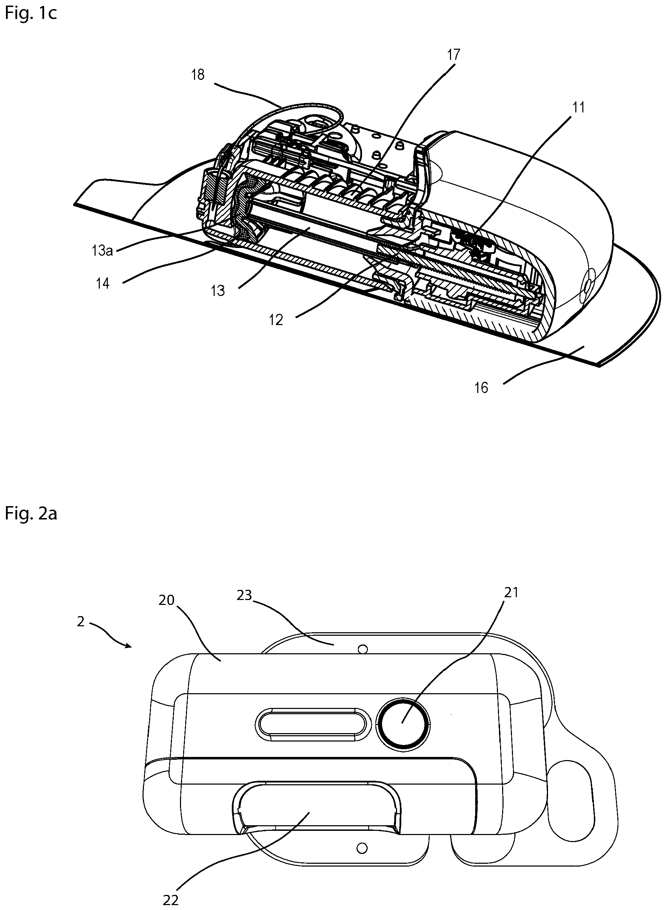

- Fig. 1a, b, c: depicts a patch pump according to the present invention;
- Fig. 1a: depicts a reusable pump unit of the patch pump in connecting position adjacent to a disposable reservoir unit;
- Fig. 1b: depicts the patch pump with pump unit and reservoir unit connected for drug delivery;
- Fig. 1c: depicts a cut across the patch pump with pump unit and reservoir unit connected for drug delivery.
- Fig. 2a, b, c: depicts a patch injector according to the present invention;
- Fig. 2a: depicts a perspective view of the patch injector;

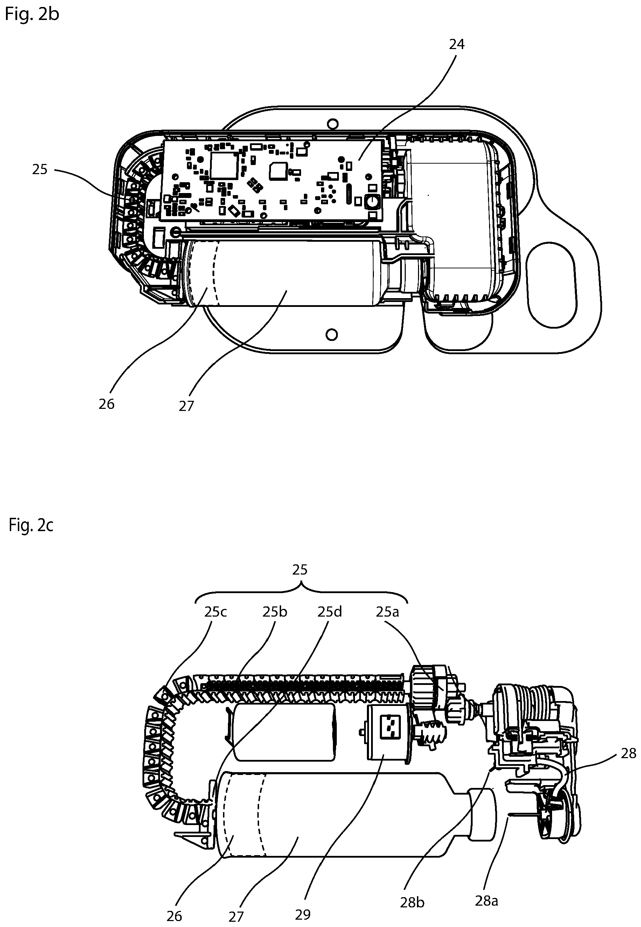

- Fig. 2b: depicts the patch injector with parts of the housing removed;
- Fig. 2c: depicts a selection of the interior of the patch injector with the motor and the actuation assembly.

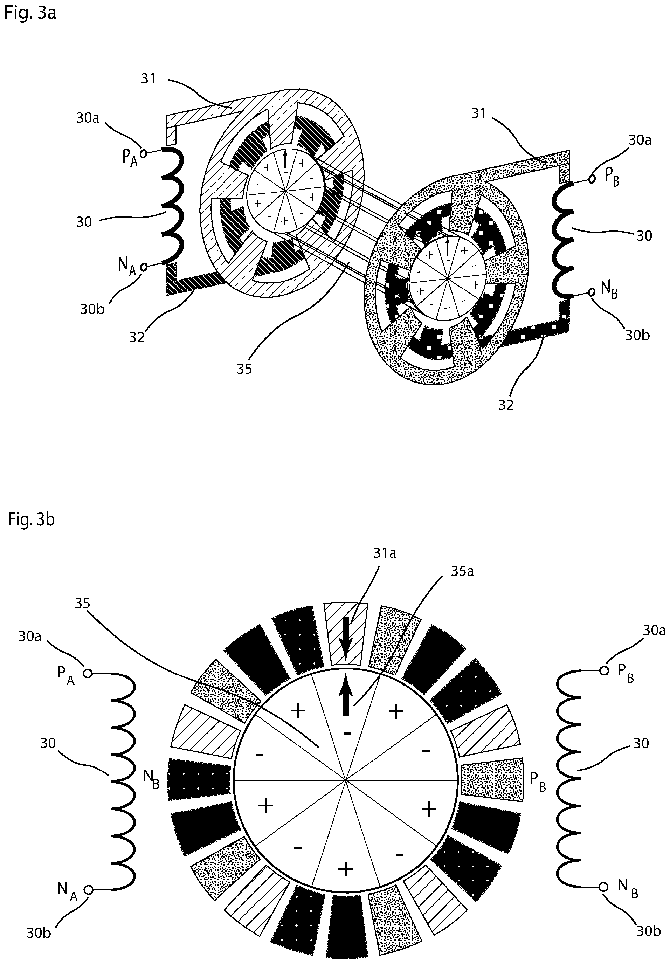

- Fig.3a, b: depicts the magnetic design of an electric motor with two phases;
- Fig. 3a: depicts a perspective view of the arrangement of magnetic poles on the stator and the rotor;
- Fig. 3b: depicts a schematic view of the arrangement of magnetic poles on a stator and a rotor to illustrate angular motor steps;

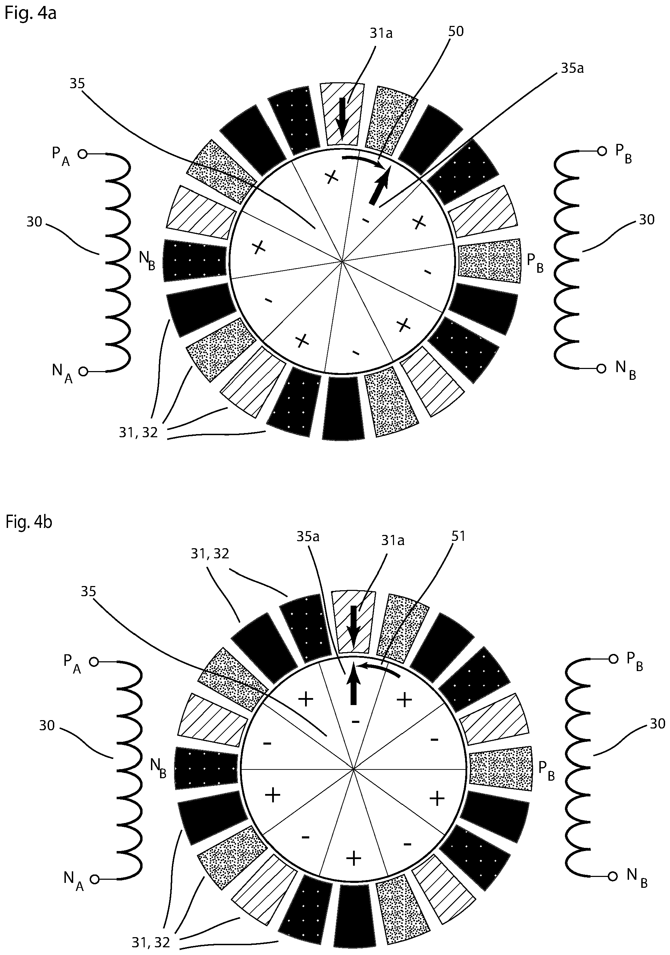

- Fig.4a, b: illustrates the process of over-revving and reversing;
- Fig. 4a: depicts the rotor after over-revving past a specific target position;
- Fig. 4b: depicts the rotor after over-revving and reversing to a specific target position;

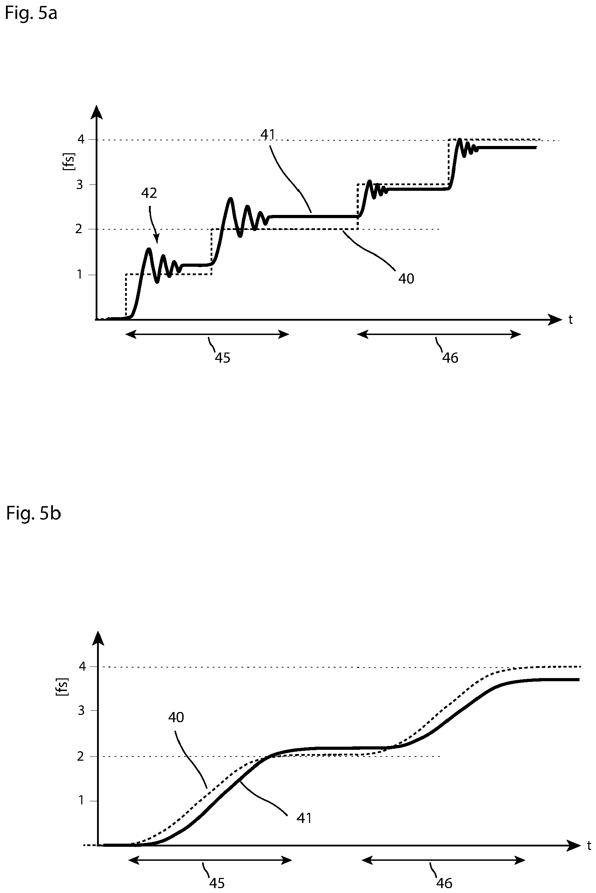

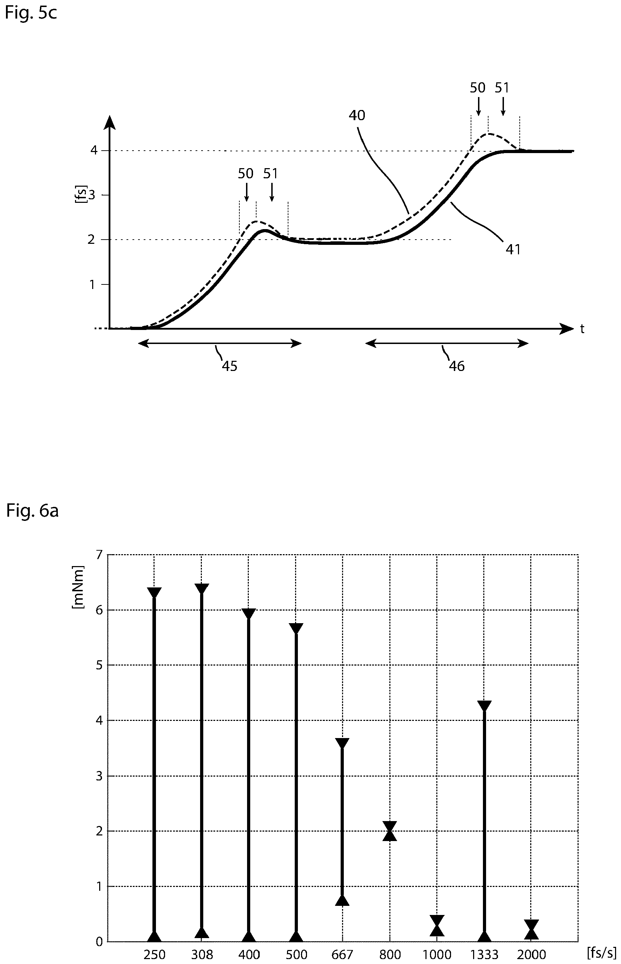

- Fig. 5a, b, c: illustrates the effect of micro-stepping and over-revving on the rotor position as evident over two subsequent delivery cycles;
- Fig. 5a: depicts the course of rotor position with full step commutation;
- Fig. 5b: depicts the course rotor position with micro-step commutation;
- Fig. 5c: depicts the course of rotor position with micro-step commutation, over-revving and reversing;

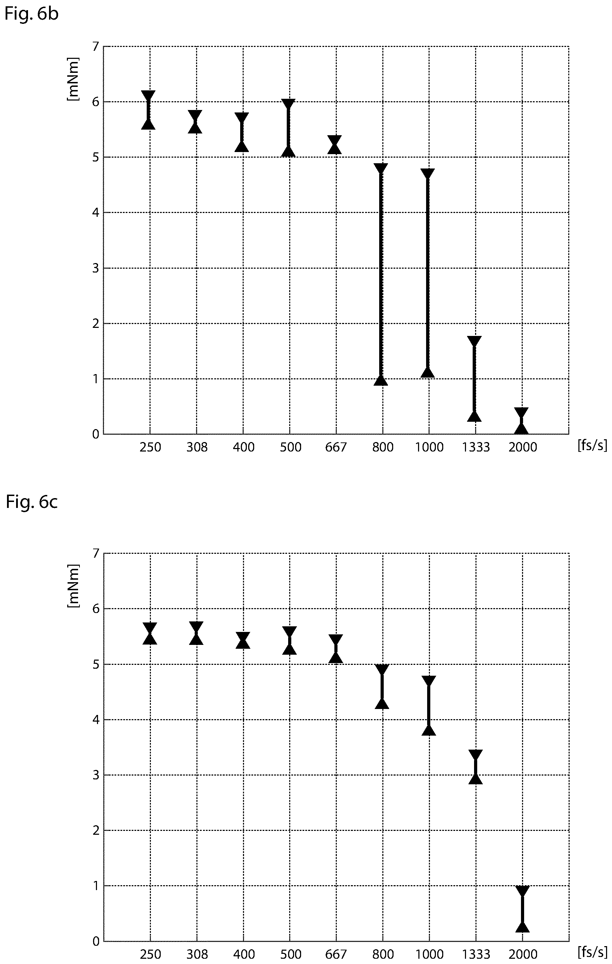

- Fig. 6a,b: illustrates the effect of micro-stepping and over-revving on the maximum step loss torque at different rotation speed;
- Fig. 6a: depicts the range of maximum step loss torque measured at increasing rotating speed with full-step commutation;
- Fig. 6b: depicts the range of maximum step loss torque measured at increasing rotating speed with micro-step commutation;
- Fig. 6c: depicts the range of maximum step loss torque measured at increasing rotating speed with micro-step commutation and over-rewing.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

Figure 1a depicts a first preferred embodiment of a drug delivery device according to the present invention. The drug delivery device is realised as a patch pump (1) attachable to the skin of a patient. A preferred application for a patch pump according to the present invention is as an infusion pump for the continuous subcutaneous infusion of insulin (CSII) in a therapy for the treatment of diabetes mellitus, or related therapies with or without automated insulin delivery (AID). The patch pump (1) comprises a reusable pump unit (10) and a disposable reservoir unit (15). The reservoir unit (15) comprises a reservoir to store the medicament and a needle assembly with a fluid path to bring the drug from the reservoir into the body of the patient. At the bottom of the reservoir unit (15) an adhesive patch assembly (16) is included to attach the patch pump (1) to the body of the patient. The pump unit (10) is releasably and sealingly connected to the reservoir unit (15) by a bayonet connection. Figure 1a shows the complete patch pump (1) with both units put together for connecting, seen from a position above the pump. In the context of the present invention, "above" or "top" refers to the side of the pump which is facing away from the patient's body when the pump is attached to the patient for drug delivery. Consequently,"bottom" or "base" refers to the side of the pump facing towards the patient's body during drug delivery. Figure 1b illustrates how the pump unit (10) is connected to the reservoir unit (15) for application. Figure 1c depicts a cut through the patch pump (1) to show the arrangement of main inner parts in the first preferred embodiment of figure 1. The pump unit (10) comprises a drive mechanism (11) for driving the plunger rod (13), an encoder to supervise the movement of the drive mechanism, a rechargeable battery and an control unit configured to control the set-up, drug delivery and supervision of the pump. The battery may be rechargeable and may be configured to be charged by a further battery in the disposable reservoir unit (15) while the drive mechanism (11) is connected to the reservoir unit (15). The drive mechanism (11) is actuated by an electric motor (not visible in this cut) and acts mechanically by transforming the rotation of the rotor (not visible in this cut) via a gearbox assembly (not visible in this cut) and a threaded rod (12) to a linear displacement of a plunger rod (13), and from there to a plunger (14) in the reservoir (17) to dispense the medical substance out of the reservoir (17). The gearbox assembly (25a) is particularly useful to realise small displacements corresponding to small amounts of drugs, where also the invention is most advantageous. The plunger rod may consist of just one threaded rod (25b), but as dealing with elasticity and mechanical play is an important advantage of the invention, alternative embodiments may include a plunger rod assembly consisting of multiple segments (25c) as shown in Fig. 2c. While basically every electric motor may be used to drive the drug delivery device, brushless DC or stepper motors are usually preferred for safety reasons. These motors require active commutation and lend themselves perfectly to realising the invention. The invention focuses on rotor movements smaller than one full rotation and is hence preferably implemented using a motor which includes at least two stator coils (30) to define at least four angular positions per 360° rotation of the rotor (35), for example a two-phase stepper motor or a two-phase brushless DC motor. A needle assembly (18) inside the reservoir unit (15) provides the fluid connection from the reservoir (17) to the exterior of the pump for application to the patient. To ensure safe handling, the patch pump (1) is manufactured, shipped, stored and prepared for use with the needle assembly (18) completely inside the enveloping shape of the pump (1). The enveloping shape is an imaginary surface enveloping the housing of the pump (1) while smoothly bridging all gaps and recesses, should any be present, to a closed shell. It is she shape of the pump (1) as perceived by the user from a distance and relevant when it comes to aspects of use like handling or wearability. Preparation of the patch pump in this embodiment includes filling the reservoir inside the disposable reservoir unit (15) from the exterior using a transfer syringe and attaching the pump to the body of the patient by means of the adhesive patch assembly (16). An inserter assembly (not shown) is included in the disposable reservoir unit (15) and configured to bring an output portion of the needle assembly (18), in particular the open distal end of the needle assembly (18), out of the enveloping shape of the pump and into the body of the patient once the pump is ready to start drug delivery. In a preferred embodiment of the patch pump (1) the needle assembly (18) includes a rigid cannula and a soft cannula, and the inserter is configured to insert the distal end of the soft cannula into the body of the patient using the rigid cannula which will subsequently be retracted for drug delivery.

Figures 2a to 2c illustrate a patch injector (2) as a second preferred embodiment of the drug delivery device according to the present invention. A preferred application for a patch injector according to the present invention is as an injector for the controlled subcutaneous delivery of a drug over a limited time. The operating time of such an injector may vary from a few seconds (e.g. injection of a highly viscous drug not suitable for injection by a classic injection pen or syringe) to a few days (e.g. delayed injection of a drug to treat possible side-effects of an ambulatory medical treatment). Patch injectors are typically prefilled and designed for single use. Consequently, the patch injector (2) typically has one single housing without a separable pump unit and reusable parts. Figure 2a shows the patch injector (2) with a housing (20) to protect the device from the environment and provide a compact, wearable shape suitable for constant use over several minutes. An adhesive patch assembly (23) is permanently attached to the housing and used to attach the patch injector to the body of a patient for application. The housing holds a reservoir (27) to contain a medical fluid, a plunger (26) movably disposed in the reservoir (27), and a fluid transport assembly (28) to connect the reservoir to the patient. In this example, the reservoir has a septum and the fluid transport assembly (28) has a needle at the input (28a) to pierce said septum and connect the fluid transport assembly (28) with the reservoir (27). At the output (28b) of the fluid transport assembly (28) is a cannula, configured to be temporarily inserted into the body of the patient for subcutaneous drug delivery. The drug delivery is effectuated by means of a brushless stepper motor (29) with a rotor (35, see Fig. 3a) operatively connected to an actuating assembly (25). The actuating assembly (25) comprises all components necessary to translate the movement of the rotor (35) to an advancement of the medical fluid. In this particular embodiment, the actuating assembly (25) is a plunger moving assembly comprising a gear box assembly (25a), a threaded rod (25b), a segmented rod (25c) and a plunger moving head (25d), configured to move the plunger (26) in the reservoir (27) forward. An electronic control unit (24), an electrical power source (not shown), hardware for electronic and/or visual and/or acoustic communication, a microprocessor, storage and software is packed into the housing and configured to control all system functions such as interacting with a user or interacting with another device involved in the drug delivery therapy, commutating the motor (29), supervising the actuation of the plunger and initiating an alarming action and/or a mitigation action if a blockage of the actuating assembly (25) has been detected. On the outside of the housing (20), a command button (21) is connected to the electronic control unit (24) to initiate drug delivery. A reservoir window (22) in the housing (20) may allow the patient to visually supervise the drug delivery by double-checking the position of the plunger (26) in the reservoir (27).

Figure 3a and 3b illustrate the brushless stepper motor used in a preferred embodiment of the invention. The motor has two driving phases A and B with two stator coils (30) radially arranged around the rotor (35), axially next to each other as shown in the exploded view of Fig. 3a. At both ends of each coil, a magnetic flux conductor (31, 32) collects the magnetic field induced by the stator coil when activated by applying an electric voltage to the driving contacts (30a, 30b) and distributes the magnetic flux to a number of protrusions, in the described embodiment five protrusions per magnetic flux conductor. The protrusions of the magnetic flux conductors (31, 32) are arranged inside the stator coil (30) alternately from both ends of the coil (30) to form a number of magnetic pole pairs used to drive the rotor (35), in the described embodiment five pole pairs per coil (30). The flux conductors of the two coils are arranged in a fixed position axially aligned around the axis of the rotor (35) and rotated by half the angle between two pole pairs, in the example given by 18 degrees. This arrangement forms a stator with two coils (30) and ten magnetic pole pairs as illustrated in the schematic view of Fig. 3b. Each coil (30) has a positive driving contact P (30a) and a positive magnetic flux conductor (31), and a negative driving contact N (30b) and a negative magnetic flux conductor (32). By cyclically applying a positive voltage to a sequence of positive and negative driving contacts, the rotor is rotated or commutated continuously - in the case of a brushless DC motor - or in a number of discrete motor steps per rotation, in the example 20 motor steps per rotation. Alternative embodiments of the invention may have a motor with a different number of driving phases, such as single phase or triple phase, may have phases with a different number of coils, such as two coils in series or multiple coils in parallel, may have more than two driving contacts per driving phase, such as a middle contact between two coils connected in series.

Common to all embodiments using a brushless stepper motor is that the control unit, to effect the delivery of a certain amount of drug, advances the rotor by a predefined number of angular steps in a delivery direction. The angular target position of the rotor is defined by the design and mechanical arrangement of the stators. Figure 3b shows a motor where the rotor is at such a target position with an arbitrary rotor mark 35a adjacent to a stator mark 31a. The marks 31a and 35a have no technical effect, they are only introduced to define an angular position of the rotor in respect to the stator. The position of magnetic poles of the stator define the possible positions of the rotor; a rotation from one rotor position to the next is called one full motor step. The power of such a motor is limited by the strenght of the magnetic field, both on the side of the rotor, and on the side of the stator. Rotor poles are typically realised using permanently magnetised materials such as ferrites; strong permanent magnets are bulky, meaning that for mobile devices, smaller magnets are preferable. Even with more expensive materials, smaller permanent magnets are typically weaker. As for the stator, a strong magnetic field is not only linked to form factor - larger stator coils provide stronger magnetic fields - but also to power consumption. The characteristics of all mechanical parts involved in drug delivery influence the movement of the rotor in response to a certain commutation. In absence of friction, inertia may cause the rotor to keep rotating after commutation, resulting in an overdose of drug delivered. Friction anywhere along the drive train may cause the rotor to lag behind, to stop short of target position, or even to lose whole steps of commutation. Sensors are typically used to supervise the mechanical effect of electric commutation, either directly at the rotor - such as magnetic hall sensors, optical encoders, back EMF voltage detectors - or anywhere else along the drive train - such as position sensors, force sensors or pressure sensors. Two kind of error conditions are linked to the movement of the rotor and typically derived or estimated by the drug delivery device from whatever sensor signals are available: underdelivery and overdelivery. Underdelivery means a risk that the patient may not get the full amount of drug as intended. Slow rotor movement or loss of steps are examples of possible sensor signals pointing towards such a condition. Overdelivery means a risk that the patient may get more than the intended amount of drug. Fast rotor movement or unexpected reading of a position sensor point towards such a condition. It is a challenge for the drug delivery device to use the sensors available to control drug delivery reliably in a variety of environmental conditions without missing critical errors, should any occur.

To perform a particular therapy and deliver a total intended amount of drug to a patient over time, both the infusion pump and the injector typically divide the drug volume into a sequence of smaller amounts, delivered intermittently over a period of multiple seconds or even several days. This is achieved by distributing a multitude of delivery cycles over the period of therapy. Each delivery cycle has a discrete amount of drug assigned to, followed by a pause to wait for the next cycle. For optimum energy efficiency, delivery cycles should be as short as possible to keep the delivery device in a low-power sleep mode for as long as possible. At the same time, drug delivery needs to be highly controllable, adding a lot of complexity to the design of drug delivery devices. For every single delivery cycle, the delivered amount of drug needs to be accurate, and supervision needs to make sure any error is immediately detected to avoid accumulation of errors and possibly hazardous behaviour of the device. This is especially true for mobile drug delivery systems, where mechanical parts are small to keep the infusor or injector wearable, and materials and tolerances lead to an inherent elasticity of the drug delivery mechanism.

It is a main challenge for the control unit to drive the motor of a delivery device during a delivery cycle at maximum speed allowing exact control of the movement, even if rotor and stator poles are small and magnetic forces involved are relatively weak, and even if the mechanical drive train behind the plunger is potentially elastic. As a key element to meet this challenge, the present invention introduces the concept of over-revving as a first important aspect. Over-revving means that the control unit does not only commute the rotor for a certain amount of steps allocated to a certain delivery cycle, but continues commutating for a predefined number of extra steps or micro-steps after reaching the target position. This concept is illustrated in Fig. 4a. The rotor (35) is shown at the end of delivery for one cycle, with the rotor mark (35a) rotated visibly past the target position marked by the stator mark (31a). Arrow (50) emphasizes the over-revving commutation. The amount of steps used for over-revving depends on the elasticity of the mechanical drive train, on the design of the gearbox - if any - and the number of steps per full rotation. Over-revving is not intended to cause any extra movement of the plunger, but only to deal with mechanical variation in the system. Assuming that the intended delivery accuracy is in the range of zero to ten motor steps, the number of over-revving steps (OFS) may hence be in the same range. In a preferred embodiment, where over-revving is combined with micro-stepping commutation, over-revving (50) is not limited to full motor steps, but may be set to any number of micro-steps corresponding to a full step range of 0 < OFS <= 10. Keeping energy efficiency in mind, a most preferred range of over-revving (50) is between 0.25 full motor steps and 1.25 full motor steps.

It is important to note here that in a preferred embodiment of the present invention, over-revving (50) is primarily defined in terms of commutation, and not in terms of physical rotor movement. In Fig. 4a, with the rotor mark (35a) actually about 1.5 steps over the target stator mark (31a), the over-revving (50) does indeed indicate the physical movement. However, if mechanical conditions vary, the mechanical movement may well differ from the exact commutation, just like the rotor position may differ from commutation during normal operation. Alternative embodiments of the present invention may focus on the physical rotation and insist on physical over-revving rather than using an over-revving commutation and let the rotor follow as friction conditions allow. The differences between commutation and effective rotor position are further explained using Fig. 5 below. To help dealing with these differences, a second important aspect of the present invention is introduced, as illustrated in Fig. 4b: reversing. Reversing means that the control unit, after commutating the motor and effectuating drug delivery for one delivery cycle, not only continues forward commutation for over-revving, but also stops and subsequently reverses commutation for the same number of over-revving steps, resulting in a stop of the rotor closer to the original target position for that particular delivery cycle. Arrow (51) illustrates the reversing - again showing a movement which is present in commutation, but may or may not be exactly replicated in the physical movement of the rotor (35). While over-revving is an active process by nature, no active commutation may be required, it may also occur naturally due to the force of permanent magnets or the mechanic inertia involved.

Figure 5 shall illustrate in more detail how the rotor position is linked to the commutation, and how the main aspects of the present invention are realised. Starting with Fig. 5a, it is clearly visible that raw full-step commutation results in a rotor position which can sometimes be rather erratic. The dotted line (40) represents the commutation, jumping from an arbitrary zero to full step 1, to full step 2 and further to steps 3 and 4. The solid line (41) shows the rotor position effectively measured using an optical encoder. For illustration purposes, two subsequent delivery cycles (45) are shown of two full steps each. No difference is visible between the target position of a delivery cycle - at step 2 and at step 4 - and full step positions. At the end of each commutation step, the mechanical characteristics of the drive mechanism causes the rotor position to oscillate (42) before settling at a certain position (41). The rotor position typically does not stay at one of the exact motor step positions; depending on inertia and friction it will be either advanced, as in delivery cycle 1 (45), or behind, as in delivery cycle 2 (46). This means that the torque at the beginning of the next delivery cycle will show a substantially higher variation, making error detection much more difficult. The less precise angular position of the rotor may further lead to early step loss under increasing friction. As well known from prior art, the stepwise movement of the rotor also increases energy consumption and is therefore not well suited for use in a mobile or wearable drug delivery device.

A first improvement to full-step commutation is shown in Fig. 5b, where a micro-stepping commutation (40) is used to bring the rotor from zero to a first target position at two full steps during delivery cycle 1 (45), and from there to a second target position at four full steps for delivery cycle 2 (46). The movement of the rotor (41) is much smoother, the oscillations (42) are gone. Consequently, the energy efficiency has improved - but the rotor position is still somewhat off target at the end of a delivery cycle, still resulting in a high variation of torque at the beginning of the next delivery cycle.

Fig.5c now illustrates how over-revving (50) and reversing (51) as important aspects of the present invention affect the difference between commutation (40) and effective rotor position (41). The rotor position is smoothly brought almost exactly to the target position of each delivery cycle. In the presence of higher friction, as shown in delivery cycle 2 (46), the over-revving (51) may be needed for extra forward commutation power, while the reverse commutation (51) does not result in a physical reverse movement of the rotor. The rotor just smoothly approaches its angular target position and stays there. The present invention hence improves the precision of rotor control for all possible frictional situations and ensures accurate drug delivery for every single delivery cycle. Most advantageously, over-revving (50) and reversing (51) according the present invention results in an almost constant torque at the beginning of each delivery cycle, allowing for a significantly improved mechanical controllability and consequently for the possibility of significantly increasing the motor speed during delivery cycles. This is explained now using Fig. 6.

The graphs in Fig. 6a, 6b and 6c show the step loss torque measured in a drug delivery device over a number of delivery cycles at stepwise increasing rotor speed. Speed was kept constant to get a set of measurements before advancing to the next level. At each speed the maximum torque achieved in each delivery cycle was determined, and data were collected for all delivery cycles where at least one full motor step was lost. As in Fig. 5, very short delivery cycles of just two full motor steps were chosen to demonstrate the huge effect of the invention for the delivery of small amounts of drug. In Fig. 6, solid vertical lines with triangle endpoints show the range of maximum step loss torque at the constant speed of 250, 308, 400 etc. full steps per second [fs/s]. The range of maximum step loss torque at constant speed is a very good indicator of the quality of motor control and ultimately of drug delivery precision. A large variation means that loss of step may occur anytime, regardless of the drive train being relaxed or compressed. A small variation means that step loss is reliably occurring at a certain torque level - the closer to the stall torque of the system the better.

Starting with full step commutation, Fig. 6a gives the ranges of maximum step loss torque at speeds from 250f/s to 2000f/s. With a few exceptions, a large variation of maximum step loss torque has been measured for almost all speeds. Step loss is occurring rather randomly, regardless of the compression of the drive train. This effect is due to the inertia of the rotor and the elasticity of the drive train. At 800f/s an incidental resonance effect of the drive train is visible.

In Fig. 6b, micro-stepping is used to commute the rotor as described in Fig. 5b. The movement of the rotor is much smoother and therefore less erratic than the movement with full-step commutation. At lower speeds up to 667f/s, step loss is only occurring at a torque of more than 5mNm. This shows that for lower speeds the drive train is typically compressed and rather well controlled, while at higher speeds an increasing number of cases is seen where the drive train is relaxed, for example because the rotor is being pushed back to its starting position during a delivery cycle. In a speed range of up to 667f/s, the movement of the rotor is sufficiently controlled to avoid very early step loss, in a range where torque levels are nowhere near the effective stall torque of the system. If any supervision parameter technically linked to loss of steps is used for detecting an error condition, the maximum speed usable with a reliable outcome is 667f/s. At higher speeds, for example at 800f/s, occlusion detection would sometimes report blockage already at 1mNm, rendering such a detection unsuitable for use in a drug delivery device.

Turning to Fig. 6c, the maximum step loss torque is measured at the same speed levels, now with a motor control according to the present invention, including micro-stepping, over-revving and active or passive reversing as described using Fig. 5. There is a significant improvement over Fig. 6a and Fig. 6b in variation of maximum step loss torque, especially for higher speeds. Even at a speed of 1333 full steps per second, the torque range is consistently narrow. The rotor movement and the compression of the drive train are much better controlled, step loss can reliably be used for error detection, providing the basis for a significant improvement in drug delivery precision, accuracy and reliability, especially for smaller amounts of drug delivered. Furthermore, the higher motor speed directly translates to improved energy efficiency.

In delivery systems with - by comparison to the embodiments described above - extended delivery cycles and reduced pauses in-between, the exact movement or inertia of the rotor does less significantly effect the performance of the device. In such a system, the effect of the invention may be reduced or at least made less relevant. For example, if no delivery cycle is shorter than 100 full steps, the loss of one full step only corresponds to an error of 1%. There, it may be suffcient to compensate for step loss rather than try and avoid it. The invention is hence most effective in a system with comparably small amounts of drug delivered over a longer period. The use of the invention may be preferable, but not limited to, drug delivery systems with intermittent drug delivery and smallest used delivery cycles in the range of zero to 100 full motor steps.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Patch pump
- 2: Patch injector
- 10: Pump unit
- 11: Drive mechanism
- 12: Threaded rod
- 13: Plunger rod
- 13a: Plunger rod cap
- 14: Plunger
- 15: Reservoir unit
- 16: Patch assembly
- 17: Reservoir
- 18: Needle assembly
- 20: Housing
- 21: Command button
- 22: Reservoir window
- 23: Patch assembly
- 24: Electronic control unit
- 25: Plunger moving assembly / Actuating assembly
- 25a: Gear box assembly
- 25b: Threaded rod
- 25c: Segmented rod
- 25d: Plunger moving head
- 26: Plunger
- 27: Reservoir
- 28: Fluid transport assembly
- 28a: Input
- 28b: Output
- 29: Electric motor
- 30: Stator coil
- 31: Positive magnetic flux conductor
- 31a: Stator reference position
- 32: Negative magnetic flux conductor
- 35: Rotor
- 35a: Rotor reference position
- 40: Rotor commutation
- 41: Rotor position
- 42: Oscillation
- 45: Delivery cycle 1
- 46: Delivery cycle 2
- 50: Over-revving
- 51: Reversing

## Claims

1. A mobile or wearable drug delivery device (1, 2) comprising
an electric motor (29) with a rotor (35) at the output;
an electronic control unit (24) configured to control the commutation or rotation of the rotor (35) and to intermittently advance an angular position of the rotor (35) by a number of steps to convey a predefined amount of drug over a plurality of delivery cycles (45);
***characterised by*** the electronic control unit (24) being adapted to over-revving (50), for each of the plurality of delivery cycles (45), the commutation or rotation of the rotor (35) by a predefined number of over-revving steps in excess of the number of steps defined for said delivery cycles (45).

2. The mobile or wearable drug delivery device according to claim 1, whereby the electronic control unit (24) is adapted to reverse (51) the commutation or rotation of the rotor (35), after over-revving (50), by substantially the same number of over-revving steps in an opposite direction.

3. The mobile or wearable drug delivery device according to claims 1 or 2, whereby the number of over-revving steps (OFS) is in the range of 0 < OFS <= 10 full motor steps.

4. The mobile or wearable drug delivery device according to any of claims 1 to 3, whereby the electronic control unit (24) includes means to measure or estimate at least one of the following supervision parameters: motor drive current, back EMF, rotor position, rotor speed, time to reach a target position, loss of steps, plunger position, drive train friction.

5. The mobile or wearable drug delivery device according to claim 4, whereby the electronic control unit (24) is adapted to compare at least one of the supervision parameters with a predefined reference value to detect an error condition.

6. The mobile or wearable drug delivery device according to claim 5, whereby the error condition includes blockage of the motor (29) or occlusion of a fluid path at an output (28b) of the drug delivery device (1, 2).

7. The mobile or wearable drug delivery device according to claim 5, whereby the error condition includes underdelivery.

8. The mobile or wearable drug delivery device according to claim 7, whereby the drug delivery device is a patch pump (1) attachable to the skin of a patient.

9. The mobile or wearable drug delivery device according to claim 7, whereby the infusion device includes a drive mechanism (11, 25), and a reservoir (17, 27) to contain a liquid drug, and a movable plunger (14, 26); and whereby the drive mechanism (11, 25) is adapted to transform the rotation of the rotor (35) into a displacement of the plunger (14, 26) to convey the drug out of the reservoir (17, 27).

10. The mobile or wearable drug delivery device according to claim 9, whereby the drive mechanism (11, 25) includes a gearbox (25a).

11. The mobile or wearable drug delivery device according to claim 9, whereby the drive mechanism (11, 25) includes a plunger rod (13, 25c) having at least one segment (25c).

12. The mobile or wearable drug delivery device according to any preceding claim, whereby the motor (29) includes at least two stator coils (30) to define at least four angular positions per 360° rotation of the rotor (35), as for example in a two-phase stepper motor or a two-phase brushless DC motor.

13. The mobile or wearable drug delivery device according to claim 12, whereby the electronic control unit (24) is adapted to commute the rotor (35) by a maximum of 100 full motor steps for a delivery cycle (45).

14. A method of controlling an electric motor (29) in a drug delivery device (1, 2), whereby the method includes over-revving (50) the rotation or commutation of the rotor (35) by a predefined number of over-revving steps as defined in claim 1.

15. The method according to claim 14, whereby the method includes reversing (51) the commutation or angular position of the rotor (35), after over-revving (50), by the same number of over-revving steps in an opposite direction.
